(19) Europäisches Patentamt
European Patent Office
Office européen des brevets

(11) Numéro de publication : **0 404 619 B1**

(12) **FASCICULE DE BREVET EUROPEEN**

(45) Date de publication du fascicule du brevet : **29.09.93 Bulletin 93/39**

(51) Int. Cl.$^5$ : **C07C 209/10**, C07C 211/48, C07C 211/52

(21) Numéro de dépôt : **90401481.8**

(22) Date de dépôt : **01.06.90**

(54) **Procédé de préparation de N-monoalkyl ou alkenyl anilines.**

(30) Priorité : **05.06.89 FR 8907388**

(43) Date de publication de la demande : **27.12.90 Bulletin 90/52**

(45) Mention de la délivrance du brevet : **29.09.93 Bulletin 93/39**

(84) Etats contractants désignés : **AT BE CH DE DK ES FR GB GR IT LI LU NL SE**

(56) Documents cités :
**EP-A- 0 205 391**
**US-A- 2 286 678**
**US-A- 3 819 708**
**US-A- 4 210 589**

(73) Titulaire : **RHONE-POULENC CHIMIE**
**25, quai Paul Doumer**
**F-92408 Courbevoie Cédex (FR)**

(72) Inventeur : **Desmurs, Jean-Roger**
**La Jonquière, Route de Ternay**
**F-69360 Communay (FR)**
Inventeur : **Kempf, Hubert**
**9, avenue de Ménival**
**F-69005 Lyon (FR)**
Inventeur : **Back-Posta, Francis**
**rue Blanche Dumont, Allée des Cyprès**
**F-69290 Craponne (FR)**

(74) Mandataire : **Dubruc, Philippe et al**
**RHONE-POULENC CHIMIE Service Brevets Chimie 25, quai Paul-Doumer**
**F-92408 Courbevoie Cédex (FR)**

**EP 0 404 619 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne un procédé de préparation de N-monoalkyl- ou alkényl anilines. Elle concerne plus particulièrement la préparation de N-monoallylanilines.

La préparation des N-monoallylanilines est particulièrement importante dans le cas de la trifluorométhylaniline car le dérivé monoallylique obtenu est un intermédiaire important dans la synthèse d'un herbicide tel que décrit dans le brevet FR 2 305 434. Selon ce brevet, pour préparer l'herbicide désiré, la N-metatrifluorométhylphényl-1 chloro-3 chlorométhyl-4 pyrrolidone-2, il est nécessaire de partir d'une trifluorométhylaniline dont un des atomes d'hydrogène est protégé par un groupe acétyl avant de procéder à l'allylation, ceci afin d'éviter la formation de produits secondaires de diallylation qui sont inutilisables.

L'industrie cherche depuis longtemps à accéder à la monoallyltrifluorométhylaniiine directement en une seule étape, au lieu des trois étapes décrites dans le brevet FR 2 305 434, avec de bons rendements calculés sur la matière première engagée la metatrifluorométhylaniline qui est un composé très onéreux que l'industrie ne veut pas perdre.

Une première solution à ce problème a été proposée dans le brevet US 4 701 560 qui décrit un procédé d'allylation de la metatrifluorométhylaniline en milieu biphasique, eau-solvant organique en présence d'une base minérale choisie parmi les carbonates ou la soude et en présence de quantités catalytiques d'une amine tertiaire quaternisable. Pour obtenir une faible quantité de produits secondaires diallyliques, il est nécessaire de limiter le taux de transformation de la metatrifluorométhylaniline et donc de travailler en présence d'un défaut d'halogénure d'allyle, il est précisé dans ce texte que le rapport metatrifluorométhylaniline à l'halogénure d'allyle est de préférence d'environ 2.

Les rendements en N-monoallylaniline calculés sur la metatrifluorométhylaniline introduite ne dépassent pas 40 % ce qui est insuffisant pour une bonne rentabilité économique du procédé.

Les réactions d'allylation sur d'autres anilines que la métatrifluorométhylaniline sont décrites par exemple dans le brevet US. 2 286 678 qui décrit notamment l'allylation de la parahydroxyaniline dans un milieu constitué d'un alcool et en présence de carbonate comme agent de neutralisation. Les rendements annoncés en N-monoallylhydroxyaniline ne dépassent pas ceux du brevet précédent et il y a en outre formation d'une quantité non négligeable de dérivés diallyliques que nous cherchons à éviter. Cette technique n'est donc pas transposable dans notre cas.

Il est également décrit dans le brevet US. 3 668 254 un procédé qui consiste à allyler l'amino-4 diphénylamine avec le dichloro- 2,3 propène en présence de triethylamine. Les rendements annoncés comme dans les deux cas précédents ne dépassent pas 40%.

En plus, la triethylamine est utilisee en quantité plus que stoechiométrique par rapport à l'halogénure d'allyle. Cette technique d'un point de vue économique est doublement peu intéressante, les rendements sont faibles et le coût des matières premières engagées est trop élevé.

Le brevet US. 3 819 708 décrit l'alkylation de paraphénylènediamines dans divers solvants en présence d'une amine tertiaire telle que la triéthylamine ou d'une base minérale comme agent de neutralisation de l'hydracide formé. Les agents alkylants qui sont décrits sont beaucoup moins réactifs que les halogénures d'allyle et le problème de dialkylation est donc beaucoup moins important. La sélectivité, c'est à dire le rendement en produit monoalkylé par rapport aux dérivés dialkylés, n'est jamais décrite.

Malgré l'existence d'une abondante littérature décrivant l'alkylation ou l'allylation de diverses anilines, aucun procédé n'a jamais permis de résoudre le problème évoqué dans la présente invention c'est à dire avoir un bon taux de transformation de l'aniline de départ et obtenir une bonne sélectivité aniline monosubstituée sur l'azote par rapport a l'aniline disubstituée.

La présente invention a permis d'atteindre cet objectif.

Elle a pour objet un procédé de préparation de N monoalkyl ou N monoalkénylanilines caractérisé en ce que l'on met en contact l'aniline et un halogénure d'alkyle ou d'alkényle en présence d'une base non quaternisable et d'un sel d'une base organique non quaternisable préparé extemporairement ou in situe.

Le sel de la base non quaternisable est de préférence un sel d'amine non quaternisable. Il est préparé par mise en contact au sein du milieu réactionnel ou préalablement d'une amine non quaternisable identique ou différente de celle servant à la neutralisation du milieu réactionnel et d'un acide choisi parmi : les acides halogènés tels que

- l'acide chlorhydrique
- l'acide bromhydrique
- l'acide sulfurique
- l'acide nitrique
- l'acide trifluoroacétique
- l'acide perchlorique

- l'acide trifluorométhanesulfonique
- l'acide phosphorique

On préfère parmi l'ensemble des acides l'acide chlorhydrique ou l'acide bromhydrique.

Les halogénures d'alkyle ou d'alkényle sont choisis parmi les composés contenant 1 à 6 atomes de carbone linéaires ou ramifiés et pouvant en outre comporter des subtituants choisis parmi les radicaux halogéno, aryle, aralkyle, halogénoarylel nitroaryle. On préfère parmi les halogénures utiliser les chlorures et les bromures et tout particulièrement les chlorures car ils sont les moins chers.

Parmi les halogénures d'alkényle la présente invention vise tout particulièrement les halogénures d'allyle et surtout les chlorures.

On peut citer parmi les agents d'alkylation :

- le chlorure d'allyle
- le bromure d'allyle
- le chlorure de benzyle
- le bromure de benzyle
- le bromure d'isopropyle
- le chlorure de crotyle
- le chloro-1 butène-2

Le procédé de la présente invention s'applique à l'ensemble des anilines, il est cependant particulièrement intéressant pour les anilines peu basiques c'est-à-dire pour les anilines présentant un pKa inférieur à 4,5.

Les anilines préférées c'est à dire présentant un pka inférieur à 4,5 sont représentées par la formule (I) suivante :

(I) 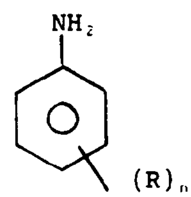

dans laquelle

- R représente :
- un halogène
- un groupe - A $C_n$ $X_{2\ n\ =\ 1}$ où X représente un halogène, A une liaison covalente, un atome d'oxygène ou de soufre
- un groupe nitro
- n est égal à 0,1 ou 2

On peut citer parmi les anilines de formule (I)

- l'aniline
- les chloroanilines
- les fluoroanilines
- les nitranilines
- les trihalogénométhylanilines
- les trihalogénométhoxyanilines
- les trihalogénométhylthioanilines

On entend par base organique non quaternisable l'ensemble des amines tertiaires présentant au moins une chaîne alkyle ramifiée et de préférence au moins deux chaînes alkyles ramifiées. On peut citer parmi ces amines à titre d'exemple :

- la diisopropylallylamine
- la diisopropyléthylamine
- la triisopropylamine
- la dicyclohexyléthylamine
- la diisobutylallylamine
- la diisopropylpropylamine

On préfère parmi l'ensemble des bases citées utiliser la diisopropyléthylamine.

L'acide est choisi de préférence parmi l'acide chlorhydrique et l'acide bromhydrique. Ainsi on préfère utiliser le chlorhydrate de la diisopropyléthylamine. La réaction est réalisée de préférence en l'absence de solvant, autre que les réactifs, la base non quaternisable pouvant servir de solvant.

Pour une meilleure mise en oeuvre de l'invention on préfère utiliser une quantité environ stoechiométrique d'halogénure d'alkyle ou d'alkényle par rapport à l'aniline.

L'acide est utilisé en quantité environ catalytique c'est à dire en quantité molaire comprise entre 0,05 et 0,2 par rapport à l'aniline.

La base, lorsqu'elle ne sert pas de solvant, est utilisée en quantité approximativement stoechiométrique par rapport à l'halogénure d'alkyle ou d'alkényle.
Elle est avantageusement utilisée selon un rapport molaire par rapport à l'halogénure d'alkyle ou d'alkényle compris entre 1 et 1,5.

La température de réaction est avantageusement comprise entre 0° et 150°. Elle variera en fonction des réactifs mis en présence surtout en fonction du pka de l'aniline et de la nature de l'halogénure. La pression réactionnelle est de préférence la pression atmosphérique. La durée de réaction varie entre une et quelques heures.

La présente invention sera plus complètement décrite à l'aide des exemples suivants qui ne doivent pas être considérés comme limitatifs de l'invention;

Exemple 1

Dans un réacteur de 30 ml sous agitation magnétique on introduit :
- meta trifluorométhylaniline = 0,497 ml (644,4 mg) ou 4 mmoles
- chlorure d'allyle = 0,326 ml (306 mg) ou 4 mmoles
- diisopropyléthylamine = 0,697 ml (517 mg) ou 4 mmoles
- diisopropyléthylamine, chlorhydrate = 66,3 mg ou 0,4 mmoles.

Le tube est chauffé pendant 4 heures à 80°C. On ajoute ensuite 0,7 ml de NaOH à 30 % (10N) et 20 ml d'eau distillée. On extrait à l'éther diisopropylique. Le dosage est réalisé par chromatographie phase gazeuse (voir tableau de résultats). Un essai comparatif (C1) est réalisé dans les mêmes conditions en l'absence de chlorhydrate de diisopropyléthylamine.

| Exemple | mTFMA | Nallyl mTFMA | N,N - diallyl mTFMA |
|---|---|---|---|
| 1 | 0,96 mmol | 2,60 mmol | 0,39 mmol |
| C1 | 2,11 mmol | 1,53 mmol | 0,13 mmol |

Exemple 2

L'exemple 1 a été reproduit mais la durée de réaction a été limitée à 2 heures.

| Exemples | mTFMA | Nallyl mTFMA | N,N - diallyl mTFMA |
|---|---|---|---|
| 2 | 2,12mmol | 1,57 mmol | 0,11 mmol |
| C2 | 3,52 mmol | 0,38 mmol | / |

Exemple 3 à 6

L'exemple 2 a été reproduit (durée de réaction 2 heures à 80°C) en variant la nature du sel de la diisopropyléthylamine ou en ajoutant uniquement un acide halogéné.

| Exemples | Nature du sel de DIPEA ou de l'acide | acide<br>----mol<br>m TFMA | mTFMA mmol | Nallyl mTFMA mmol | Diallyl mTFMA mmol |
|---|---|---|---|---|---|
| 3 | DIPEA/HCl | 4,7% | 2,78 | 1,08 | 0,06 |
| 4 | DIPEA/HCl | 15% | 2,03 | 1,61 | 0,13 |
| 5 | HCl(37%) dans l'eau | 10.2 % | 2,19 | 1,52 | 0,12 |
| 6 | HBr (47%) dans l'eau | 9,9 % | 1,52 | 1,96 | 0,22 |

Exemple 7

Dans un réacteur en charge

| | |
|---|---|
| métatrifluorométhylaniline= | = 3,25 moles (523,6g) |
| diisopropylethylamine | = 3,25 moles (420,1 g) |
| on coule le chlorure d'allyle | = 2,925 moles (223,8 g) le réacteur étant maintenu à 80°C |

| | coulée chlorure | | | | Maintien | | | |
|---|---|---|---|---|---|---|---|---|
| | 30mn | 1h00 | 1h30 | 2h00 | 30mn | 1h00 | 1h30 | 2h00 |
| TT% chorure allyle | | | 1,2 | | 26,2 | 44,7 | 66,1 | 76,4 |
| TT% m.TFMA | 2,7 | 4,4 | 5,9 | 15,9 | 23,7 | 38,8 | 52,5 | 60,3 |
| RR% N.ALLYL | 0,8 | 2,2 | 5,0 | 10,5 | 21,7 | 36,4 | 47,6 | 54,6 |
| RR % Di-Allyl | 0 | 0 | 0,07 | 0,4 | 1,0 | 2,4 | 4,0 | 5,0 |
| Sélectivité % | 100 | 100 | 98,8 | 96,6 | 95,4 | 93,9 | 92,3 | 91,7 |

Le même essai est réalisé en ajoutant 53,9 g (0,325 moles) de chlorhydrate de diisopropylamine

| | coulée chlorure | | | | Maintien | | | |
|---|---|---|---|---|---|---|---|---|
| | 30mn | 1h00 | 1h30 | 2h00 | 30mn | 1h00 | 1h30 | 2h00 |
| TT% chlorure Allyle | | 19,2 | 24,0 | 50,1 | 67,6 | 77,0 | 85,4 | 89,1 |
| TT% m-TFMA | 7,6 | 10,9 | 24,6 | 40,0 | 53,7 | 63,6 | 68,0 | 70,8 |
| RR% N-Allyl | 2,2 | 8,6 | 19,5 | 36,6 | 49,3 | 55,8 | 61,0 | 64,0 |
| RR% DI-ALLYL | 0 | 0,1 | 0,6 | 2,0 | 3,7 | 5,1 | 6,1 | 6,8 |
| Sélectivité % | 100 | 98,2 | 96,9 | 94,8 | 93,0 | 91,6 | 90,9 | 90,4 |

## Revendications

1. Procédé de préparation de N monoalkylanilines ou N monoalkénylanilines caractérisé en ce que l'on met en présence une aniline avec un halogénure d'alkyle ou d'alkényle en présence d'une base organique non quaternisable et d'un sel d'une base organique non quaternisable.

2. Procédé selon la revendication 1 caractérisé en ce que l'aniline est la métatrifluorométhylaniline.

3. Procédé selon la revendication 1 caractérise en ce que l'halogénure d'alkyle ou d'alkényle contient 1 à 6 atomes de carbone et est de préférence un halogènure d'allyle.

4. Procédé selon la revendication 1 caractérise en ce que le sel de la base organique non quaternisable est obtenu par mise en contact de la dite base et d'un acide choisi parmi les acides halogènés, l'acide sulfurique l'acide nitrique, l'acide trifluoroacétique, l'acide perchlorique, l'acide trifluorométhane sulfonique, l'acide phosphorique.

5. Procédé selon la revendication 4 caractérise en ce que l'halogénure d'allyle est le chlorure d'allyle.

6. Procédé selon la revendication 1 caractérisé en ce que la base non quaternisable est la diisopropyléthylamine.

7. Procédé selon la revendication 1 caractérisé en ce que le sel d'une base organique non quaternisable est le chlorhydrate ou le bromhydrate de la diisopropylethylamine.

8. Procédé selon la revendication 1 caractérisé en ce que le rapport molaire entre l'halogénure d'alkyle ou d'alkényle et l'aniline est compris entre 0,9 et 1,2.

9. Procédé selon la revendication 1 caractérise en ce que le rapport molaire entre la base non quaternisable et l'halogénure d'alkyle ou d'alkényle est compris entre 1 et 1,5.

10. Procédé selon la revendication 1 caractérisé en ce que le rapport molaire entre l'acide et l'aniline est compris entre 0,05 et 0,20.

## Patentansprüche

1. Verfahren zur Herstellung von N-Monoalkylanilinen oder N-Monoalkenylanilinen, dadurch gekennzeichnet, daß man ein Anilin mit einem Alkylhalogenid oder einem Alkenylhalogenid in Gegenwart einer organischen nicht-quaternisierbaren Base und eines Salzes einer organischen nicht-quaternisierbaren Base zusammenbringt.

2. Verfahren nach Anspruch 1, dadurch gekennzeichnet, daß das Anilin m-Trifluormethylanilin ist.

3. Verfahren nach Anspruch 1, dadurch gekennzeichnet, daß das Alkylhalogenid oder Alkenylhalogenid 1 bis 6 Kohlenstoffatome enthält und vorzugsweise ein Allylhalogenid ist.

4. Verfahren nach Anspruch 1, dadurch gekennzeichnet, daß das Salz der organischen nicht-quaternisierbaren Base erhalten wird, indem die Base mit einer Säure, ausgewählt unter den halogenierten Säuren, Schwefelsäure, Salpetersäure, Trifluoressigsäure, Perchlorsäure, Trifluormethansulfonsäure und Phorphorsäure, in Berührung gebracht wird.

5. Verfahren nach Anspruch 4, dadurch gekennzeichnet, daß das Allylhalogenid Allylchlorid ist.

6. Verfahren nach Anspruch 1, dadurch gekennzeichnet, daß die nicht-quaternisierbare Base Diisopropylethylamin ist.

7. Verfahren nach Anspruch 1, dadurch gekennzeichnet, daß das Salz einer organischen nicht-quaternisierbaren Base das Chlorhydrat oder Bromhydrat von Diisopropylethylamin ist.

8. Verfahren nach Anspruch 1, dadurch gekennzeichnet, daß das Molverhältnis zwischen Alkylhalogenid oder Alkenylhalogenid und Anilin im Bereich von 0,9 bis 1,2 liegt.

9. Verfahren nach Anspruch 1, dadurch gekennzeichnet, daß das Molverhältnis zwischen nicht-quaternisierbarer Base und Alkylhalogenid oder Alkenylhalogenid im Bereich von 1 bis 1,5 liegt.

10. Verfahren nach Anspruch 1, dadurch gekennzeichnet, daß das Molverhältnis zwischen Säure und Anilin im Bereich von 0,05 und 0,20 liegt.

## Claims

1. Process for the preparation of N-monoalkyl anilines or N-monoalkenyl anilines, characterized in that an aniline is brought together with an alkyl or alkenyl halide in the presence of a non-quaternizable organic base and a salt of a non-quaternizable organic base.

2. Process according to claim 1, characterized in that the aniline is metatrifluoromethyl aniline.

3. Process according to claim 1, characterized in that the alkyl or alkenyl halide contains 1 to 6 carbon atoms and preferably an allyl halide.

4. Process according to claim 1, characterized in that the salt of the non-quaternizable organic base is obtained by contacting said base and an acid chosen from among halo acids, sulphuric acid, nitric acid, trifluoroacetic acid, perchloric acid, trifluoromethane-sulphonic acid and phosphoric acid.

5. Process according to claim 4, characterized in that the allyl halide is allyl chloride.

6. Process according to claim 1, characterized in that the non-quaternizable base is diisopropyl ethyl amine.

7. Process according to claim 1, characterized in that the salt of a non-quaternizable organic base is diisopropyl ethyl amine hydrochloride or hydrobromide.

8. Process according to claim 1, characterized in that the alkyl or alkenyl halide to aniline molar ratio is between 0.9 and 1.2.

9. Process according to claim 1, characterized in that the molar ratio between the non-quaternizable base and the alkyl or alkenyl halide is between 1 and 1.5.

10. Process according to claim 1, characterized in that the molar ratio between the acid and the aniline is between 0.05 and 0.20.